# EUROPÄISCHE PATENTSCHRIFT

(11) **EP 0 430 951 B1**
(45) Veröffentlichungstag und Bekanntmachung des Hinweises auf die Patenterteilung: **01.09.1993**
(21) Anmeldenummer: 89907138.5
(22) Anmeldetag: 22.06.1989
(51) Int. Cl.: A61H 23/00, A61N 2/00

(54) **MAGNETISCHE MASSAGETHERAPIE-VORRICHTUNG**
MAGNETIC MASSAGE-THERAPY DEVICE
DISPOSITIF DE MASSAGE THERAPEUTIQUE MAGNETIQUE

(30) Priorität: 22.06.1988 YU 1189/88
(43) Veröffentlichungstag der Anmeldung: 12.06.1991
(73) Patentinhaber: Urban, Pavle, Dr., D-33181 Wünnenberg (DE); Susic, Dragan, 11251 Beograd-Ostruznica (YU)
(72) Erfinder: SUSIC, Dragan, YU-11251 Beograd-Ostruznica (YU)
(74) Vertreter: Hanewinkel, Lorenz, Dipl.-Phys.
(86) Internationale Anmeldenummer: EP8900689
(87) Internationale Veröffentlichungsnummer: WO8912433

(56) Entgegenhaltungen:
- EP-A- 0 005 713
- DE-A- 2 707 574
- DE-A- 3 335 018
- FR-A- 2 139 720
- FR-A- 2 585 579

## Beschreibung

Die Erfindung betrifft eine Behandlungsvorrichtung für einen menschlichen oder tierischen Körper oder Körperteil mit einer Feldspulenanordnung, die mit einem netzgespeisten, niederfrequenten Impulsstromgenerator verbunden ist und in der ein Magnetplättchen elastisch gehalten angeordnet ist, so daß es in dem niederfrequenten Magnetfeld der Feldspulenanordnung oszilliert.

Eine derartige Behandlungsvorrichtung ist aus der DE-A-3 335 018 vorbekannt. Diese Vorrichtung weist jedoch jeweils zwei den Patientenkörper beaufschlagende Feldspulen auf, die an jeweils einem Impulsstromgenerator angeschlossen sind. Diese Impulsgeneratoren arbeiten mit sehr verschiedenen Frequenzen, nämlich einer niedrigen Grundfrequenz und einer hohen Modulationsfrequenz von über 10 kHz. Dies ist ein hoher Aufwand und erfordert jeweils eine geeignete Abstimmung der Einstellung der beiden Generatoren auf jeweils vorliegende Behandlungserfordernisse. Das Permanentmagnetplättchen ist in dem Feldbereich der beiden Feldspulen beweglich gehalten und übt durch seine Oszillation nur eine elektromagnetische Wirkung auf den beabstandeten Körper aus.

Weiterhin ist aus der FR-A-2 585 579 eine Behandlungsvorrichtung bekannt, deren Spulen nur einseitig mit dem Frequenzgenerator verbunden sind und miteinander isoliert und zum Körper hin nicht isoliert sind, sondern durch einen Schutzschleier nur vor direkter Berührung geschützt sind. Vielmehr besteht hierbei über die spulenartige Leiteranordnung durch die nicht völlig isolierende Umhüllung eine elektrisch leitende Verbindung zum Körper des Patienten, den bei Anlegen zweier solcher Elektroden ein Mikrostrom durchfließt, der aus einer Mikrobatterie geliefert wird, wobei jede der Elektroden an einen Pol des Frequenzgenerators angechlossen ist. Die Permanentmagnete sind in dem Innenbereich der spulenartigen Leiteranordnungen gehalten und nicht unmittelbar mit dem Körper in Kontakt. Der Mikrostrom - ein höherer wird von einem Körper nicht ertragen - durchfließt das Leitergebilde nur beschränkt da er über den Leiter verteilt diesen seitlich verläßt. Ein magnetiches Wechselfeld, das in der Lage wäre, eine Schwingungsbewegung der Permanentmagnete zu bewirken, vermag ein solcher Mikrostrom nicht zu erzeugen. Auch würde sich eine mechanische Magnetschwingung nur beschränkt durch die umgebende Leiteranordnung hindurch auf den Körper übertragen. Weiterhin ist der Körper außenseitig zum spulenartigen Leiter angeordnet, so daß nur ein geringer Bruchteil des sehr schwachen durch den Mikrostrom erzeugten magnetischen Feldes den Körper trifft.

Weiterhin ist aus der EP-A-0 005 713 eine Magnetspulenanordnung bekannt, die von Niederfrequenzstrom gespeist ist und in der ein menschlicher Körper anzuordnen ist, wobei an dem Körper ein hochpermeables Plättchen angeordnet wird, das das niederfrequente Magnetfeld konzentriert.

Die Aufgabe der Erfindung ist es, die eingangs genannte Vorrichtung zu verbessern und eine Erhöhung der Wirksamkeit der Niederfrequenztherapie und eine Erweiterung von deren Anwendungsgebiet zu erbringen.

Die Lösung der Aufgabe besteht darin, daß mehrere Magnetplättchen gegeneinander verschwenkbar in einer Magnetdecke oder einer körpergerecht gestalteten Magnetschürze gehalten sind und zwischen den Magnetplättchen elastisches Material eingelagert ist, durch das Nähte hindurchgenäht sind, und daß die Feldspulenanordnung aus nur einer hohlzylindrischen oder abgeflacht-hohlzylindrischen Feldspule oder aus mehreren Spulenabschnitten besteht, die nebeneinander angeordnet sind und an den einzigen Impulsstromgenerator gleichsinnig wirkend angeschlossen sind.

Nach der Erfindung ist der Körper oder Körperteil in der Spulenanordnung anzuordnen und die schwenkbaren Magnetplättchen sind mit der Decke oder Schürze unmittelbar an dem Körper oder Körperteil anzuordnen. Die Spule aus isolierten Leitern ist jeweils mit ihren beiden Enden an komplementäre Anschlüsse der niederfrequenten Stromquelle angeschlossen, die eingangsseitig netzgespeist ist, wobei der niederfrequente Stromfluß so bemessen ist, daß die Magnetplättchen in dem niederfrequenten Magnetfeld der Feldspulen bildenden Spulen den Körper massierende Vibrationen ausführen.

Vorteilhafte Ausgestaltungen sind in den Unteransprüchen angegeben.

Die erfinderische Vorrichtung ist sehr nützlich in der physikalischen Medizin und zur Rehabilitation von Personen. Diese Neuerung erbringt eine Verbesserung des bekannten Verfahrens der Anwendung eines niederfrequenten elektromagnetischen Feldes, Niederfrequenztherapie genannt. Die Magnetplättchen bestehen vorteilhaft aus Permanentmagnetmaterial und sind in einer Decke oder Bandage gegeneinander beabstandet und gegeneinander schwenkbar gehalten, so daß sie auf dem Behandlungsobjekt einfach angeordnet und gehalten werden können, jedoch in dem elektromagnetischen Feld durch die Interaktion der Magnetfelder Schwenkbewegungen ausführen, welche ergänzend zu der therapeutischen Wirkung der Magnetfelder einen therpeutischen Massageeffekt vorteilhaft erbringen.

In einer vorteilhaften Ausgestaltung der Gesamtvorrichtung ist die Spulenanordnung, die der Erzeugung des Niederfrequenzfeldes dient und das Behandlungsobjekt und die Permanentmagnetanordnung, d.h. die Magnetträgerdecke oder -bandage, umschließt, aus biegsamen Spulen besteht, wodurch der Transport und die Handhabung der Anordnung vereinfacht ist.

Eine vorteilhafte Ausgestaltung der Vorrichtung ist von einem Niederfrequenz-Impulsgenerator mit vorgebbarer Frequenz und Amplitude gespeist.

Medizinisch-wissenschaftliche Untersuchungen mit der neuartigen Vorrichtung erwiesen deren vorteilhafte Wirkungen gegenüber der vorbekannten. Die Versuche an Menschen und Tiere erwiesen eine wesentlich höhere Wirksamkeit bei der Behandlung von posttraumatischen Zuständen im Vergleich zur vorbekannten reinen Niederfrequneztherapie. Die Versuche wurden mti einem elektromagnetischen Niederfrequenz-Impulsgeber in zwei Ausführungen vorgenommen und zwar: erstens mit Erzeugung von Impulsen einer Frequenz, die in einem Bereich zwischen 0,5 bis 30 Hz vorgebbar war, und deren Impulse aus Wellen einer sinusförmigen oder halbsinusförmigen Gestalt mit einer Grundfrequenz von 60 Hz bestanden, und zweitens mit Rechteckimpulsen mit einer vorgebbaren Frequenz im Frequenzbereich zwischen 1 - 1000 Hz. Die Feldspulenanordnung war bei den Versuchen in bekannter Form ausgestaltet.

Teil der neuarigen Vorrichtung ist eine besonders ausgestaltete Magnet-Decke. Sie besteht aus vielen kleinen Permanentmagneten, die in bestimmter Weise orientiert und in bestimmter Weise voneinander getrennt, zueinander angeordnet sind. Hiermit entsteht in einem elektromagnetischen Niederfrequenzfeld eine sehr feine Vibration der starren, beweglich gelagerten Magnete, die eine Mikromassage der damit bedeckten Gefäße bewirkt. Der Haupteffekt dieser Mikromassage ist eine Erweiterung der kleinen Blutgefäße und eine Verbesserung des Flusses im lymphatischen System, also eine verbesserte Lymphdrainage. Darüberhinaus erbringt diese Massageart einen Erholungseffekt auf die betroffene Skelettmuskulatur. Diese Effekte überlagern die bekannten positiven Effekte der Magnet- und der Niederfrequenztherapie und erbringen einen synergistischen Effekt. Der Massageeffekt ist bereits nach wenigen Minuten spürbar, und die dadurch auftretende Entspannung und erhöhte Durchblutung ermöglicht, daß die nur langzeitlich wirkend spürbare Niederfrequenztherapie ihre Wirkung besser entfaltet.

Vorteilhaft hat sich gezeigt, daß mit den Permanentmagneten ausgerüstete Bandagen, die zur Pressur von Gliedmaßen mit Verletzungen, z.B. Zerrungen oder Muskelrissen, dienen, wenn sie in dem Niederfrequenzfeld zur Massagevibration erregt wurden, den bei Bandagen üblichen nachteiligen Lymph- und Blutstau in dem herzfernen Extremitätenabschnitt hinter der Bandage vermeiden, so daß eine schnellere und schmerz- und nachbehandlungsfreie Heilung der Verletzung bewirkt wurde.

Vorteilhafte Ausführungsformen und Applikationen sind in den Figuren 1 bis 9 dargestellt.
- Fig. 1: zeigt eine Schaltung eines Impulsgenerators der Vorrichtung;
- Fig. 2: zeigt einen Ausschnitt einer Magnetanordnung in einer Decke in Aufsicht;
- Fig. 2a: zeigt einenk Querschnitt zu Fig. 2;
- Fig. 3: zeigt eine schürzenarige Decke mit einer weiteren Magnetanordnung;
- Fig. 4: zeigt eine Feldspulenanordnung teilweise geöffnet teilweise schematisch in Aufsicht;
- Fig. 4a: zeigt einen Querschnitt zu Fig. 3;
- Fig. 5: zeigt eine elektrische Schaltung der Feldspulenanordnung zu Fig. 4;
- Fig. 6: zeigt eine weitere Feldspulenanordnung;
- Fig. 6a: zeigt einen Querschnitt zu Fig. 6 im vergrößerten Ausschnitt;
- Fig. 6b: zeigt einen Querschnitt zu Fig. 6;
- Fig. 7: zeigt eine elektrische Schaltung der Feldspulenanordnung zu Fig. 6;
- Fig. 8: zeigt ein Blockschaltbild einer Gesamtanordnung;
- Fig. 9: zeigt verschiedene Anordnungen der Vorrichtung an
- a - d: einem Patienten.

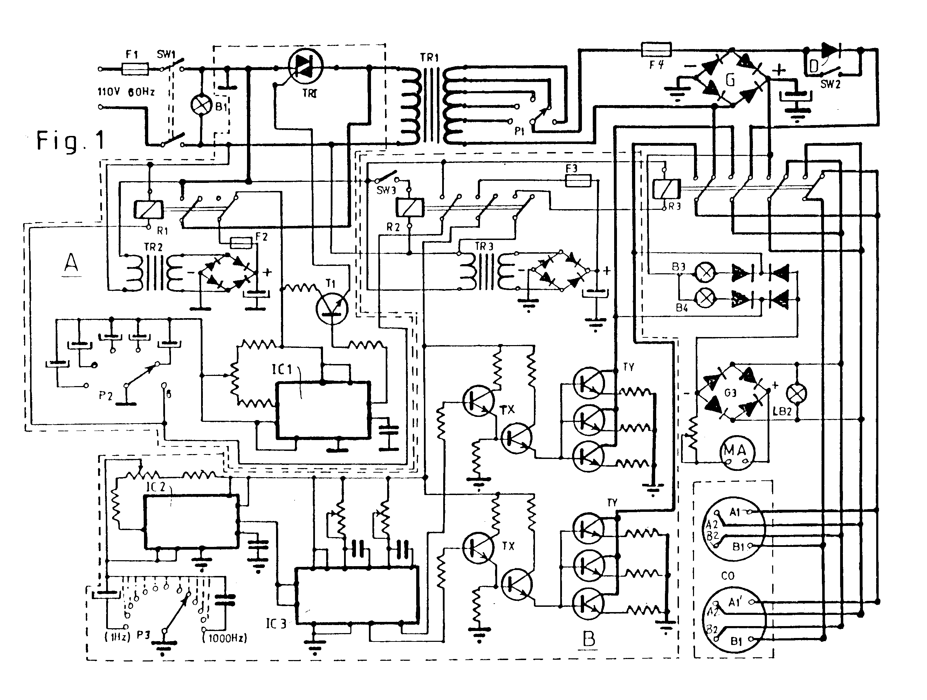

Fig. 1 zeigt ein Schaltbild des elektrischen Impulsgenerators. Dieser wird vom Wechselstromnetz (60 Hz, 110 V) gespeist, wenn der Schalter (SWA) eingeschaltet ist. Die Absicherung geschieht über die Sicherung (F1), und der Betrieb ist durch die Kontrolleuchte (B1) angezeigt. Der Betreibsartenschalter (SW3) dient der Umschaltung zwischen einer Betriebsart, bei der mit Hilfe der Netzfrequenz gearbeitet wird, wobei in seiner Aus-Stellung Signale einer Impulsfrequenz zwischen 0,5 - 30 Hz erzeugt werden und Signale mit sinusförmigem oder halbsinusförmigem Verlauf entstehen. Die Stromversorgung des ersten Oszillators (IC1) erfolgt über den Netztransformator (TR2), einen Netzgleichrichter, eine zweite Sicherung (F2) und einen Relaiskontakt eines ersten Relais (R1). Dann erzeugt der integrierte erste Oszillator (IC1), abhangig von den mit einem Frequenz-Bereichsschalter (P2) angeschalteten Kapazitäten, eine jeweils vorgegebene Frequenz zwischen 0,5 und 30 Hz. Das Oszillatorsignal verstärkt der Transistor (T1), dessen Signal einen Triac (TRI) ansteuert, der das Wechselspannungseingangssignal entsprechend getaktet einem Signaltransformator (TR1) zuführt. Dessen Sekundärwicklungen werden mit dem Leistungswahlschalter (P1) ausgewählt, und das ausgewählte Signal wird über einen Schalter (SW2), je nach dessen Stellung direkt oder über einen Halbwellengleichrichter (D), dem Ausgang zugeführt. Es werden demgemäß entweder Voll- oder Halbwellen-Ausgangssignale periodisch gemäß der Oszillatorfrequenz erzeugt. Gleichgerichtet und gefiltert ist das Ausgangssignal, das an den Steckerbuchsen (CO) abgegeben wird, einem Pegelmesser (MA) zugeführt Dessen Skala ist vorteilhaft entsprechend der Magentfeldstarke der Feldspule kalibriert. Eine Kontrolleuchte (LB2) zeigt den Betriebszustand. Wenn der Bereichsschalter (P2) in einer besonderen Endstellung (6) ist, ist das erste Relais (R1) umgeschaltet, wodurch der erste Oszillaotr (IC1) abgeschaltet ist und die Netzfrequenz direkt auf den Transformator (TR1) geführt ist, die dann gemäß der Schalterstellung direkt oder als Halbwellen ausgegeben wird.

Wenn der Betriebsartenschalter (SW3) sich in der Ein-Stellung befindet, ist der, in einem gestrichelt umrandeten Bereich gezeigte, zweite Generator (B) betrieben anstelle des im anderen gestrichelt umrandeten Bereich befindliche, bereits beschriebene, erste Generator (A). Der zweite Generator (B) liefert Rechteckimpulse mit, je nach Einstellung, einer Frequenz zwischen 1 bis 1000 Hz. Drei Relais (R1, - R3) sind dann eingeschaltet. Der zweite integrierte Oszillator (IC2) wird von einem Transformator (TR3) über einen Gleichrichter und eine Sicherung (F3) gespeist. Der zweite Oszillator (IC2) wird über die mit dem Wahlschalter (P3) jeweils angeschlossene Kapazitat in einem Bereich zwischen 1 und 1000 Hz festgelegt. Das Oszillatorsignal ist einem monostabilen Impulsgeber (IC3) zugeführt, der mit entsprechender Frequenz komplementäre Rechteckimpulse abgibt, die in Darlinston-Transistorpaaren (TS) verstärkt werden, deren Ausgangssingale zwei elektronische Schalter- oder Thyristorgruppen (TY) aus parallelen Hochleistungsthyristoren zugeführt ist. Die Gleichspannungsversorgung dieser Thyristoren (TY) erfolgt über einen Gleichrichter (G), der dem Leistungswahlschalter (P1) nachgeschaltet ist. Betriebsarten-Anzeigelampen (B3, B4) sind jeweils den beiden Thyristorgruppen (TY) nachgeschaltet deren Ausgange auf Kontakte der Steckerbuchsen (CO) geführt sind, in die das Kabel für Feldspulen zu stecken sind.

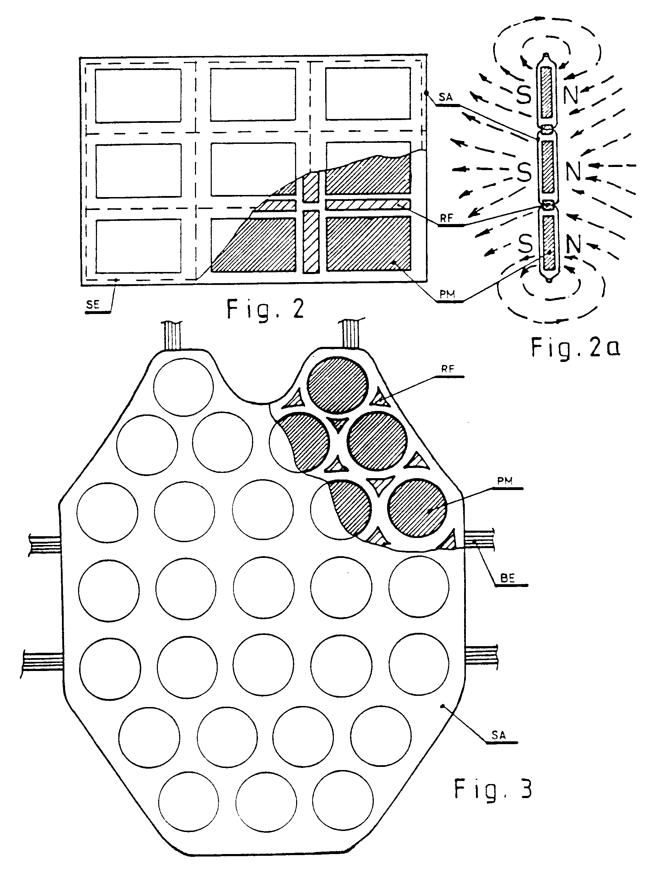

Fig. 2 und 2a zeigen einen Ausschnitt einer Aufsicht und einen Querschnitt einer Magnet-Decke. Diese besteht aus kleinen Permanentmagneten (PM). Diese können unterschiedliche Größen und Gestalt haben. Die Zahl der Magnete hängt von der Größe der Decke ab. Sie sind in einer textilen Gewebehülle (SA), z.B. aus Leinen, nebeneinander gehalten, wobei zwischen ihnen elastisches Füllmaterial (RF), z.B. aus Gummi, eingelagert, das mit der Gewebehülle (SA) vernäht ist, und zwar entlang den Randern der Magnete, wie die gestrichelt gezeichnete Naht (SE) zeigt. Dieser Aufbau der Magnet-Decke hat eine Rechteckraster-Struktur. Alle Magnete sind in gleicher Richtung orientiert, wie Fig. 2a zeigt, und ihre magnetische Achse steht senkrecht zur Erstreckung der Decke.

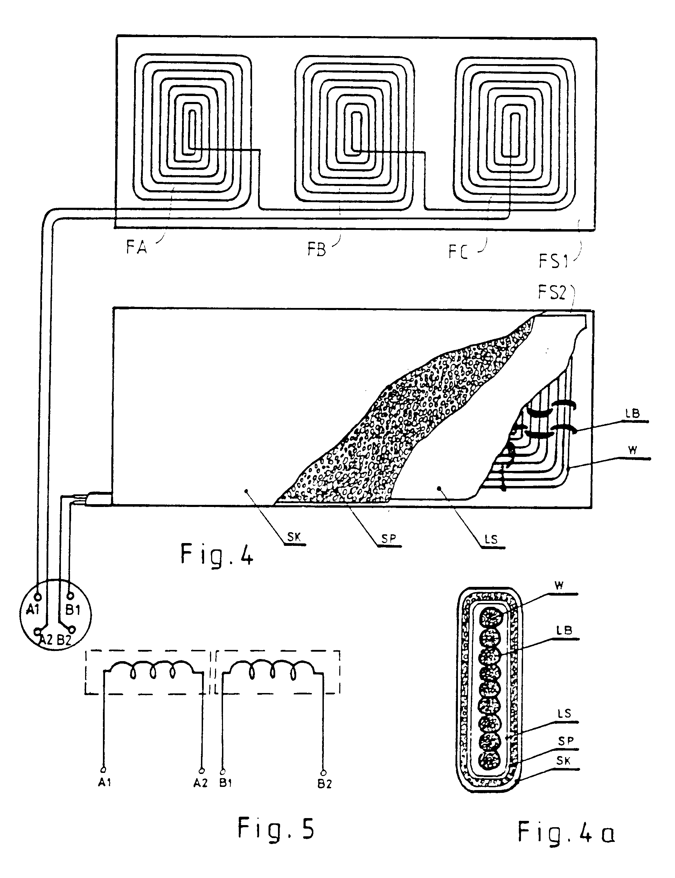

Fig. 3 zeigt eine alternative Anordnung der Magnete (PM) in Form einer Magnet-Schürze, die mit vielen runden Magnetplättchen gefüllt ist, um die herum genäht ist. Die Zwischenräume zwischen ihnen ist mit Gummit (RF) gefüllt. Seitlich sind Bänder (BE) an der Schürze befestigt, die zu deren Befestigung an einem Patientenkörper dienen.

Die Magnet-Decke oder -Schürze erzeugen die Mikromassage in dem elektromagnetischen Wechselfeld. Wird dieses eingeschaltet, so oszillieren die Permanentmagnete durch die auftretenden wechselnden magnetisch-mechanischen Kräfte, die eine zarte Massage des abgedeckten Körperteils bewirken. Hierdurch wird eine wohltuende Wirkung auf lebendes Gewebe, z.B. eine verbesserte Mirkozirkulation und eine Schmerzbehebung, ausgeübt.

Fig. 4 zeigt eine erste, flache, flexible Feldspulenanordnung der Vorrichtung, die in verschiedenen Größen herzustellen ist und aus vielen nebeneinanderliegenden, jeweils als rechteckige Spiralen aufgebauten, Leiterbündel-Abschnitten(FA, FB, FC) besteht. Eine Gesamtspulenanordnung ist vorzugsweise aus zwei getrennten Feldsoulandordnungen (FS1, FS2) gebildet.

Fig. 4a zeigt einen Querschnitt und Fig. 4 einen teilweise geöffneten Bereich der Feldspulenanordnung. Die flexiblen, isolierten Leiterbündel (W), die die Spulenabschnitte bilden, sind durch eine Ummantelung (LB) miteinander verbunden und nebeneinander gehalten, so daß sie die flache rechteckige Spirale bilden. Die drei Spulenabschnitte sind hintereinander geschaltet. Sie sind zwischen einer beidseitigen Textilabdeckung (LS) eingeschlossen und gehalten.

Vorteilhaft ist um die Textilabdeckung (LS) eine dünne Schicht aus Schaumstoff (SP) und eine Kunststoffabdeckung (SK) herumgelegt.

Die beiden Spulenanordnungen (FS1, FS2) werden so bestromt und übereinander oder nebeneinander angeordnet, daß sich ihre Magnetfelder addierend überlagern, wie Fig. 5 schematisch zeigt. Die Anschlüsse (A1, A2; B1, B2) sind an die Steckerbuchse (CO), Fig. 1, geführt.

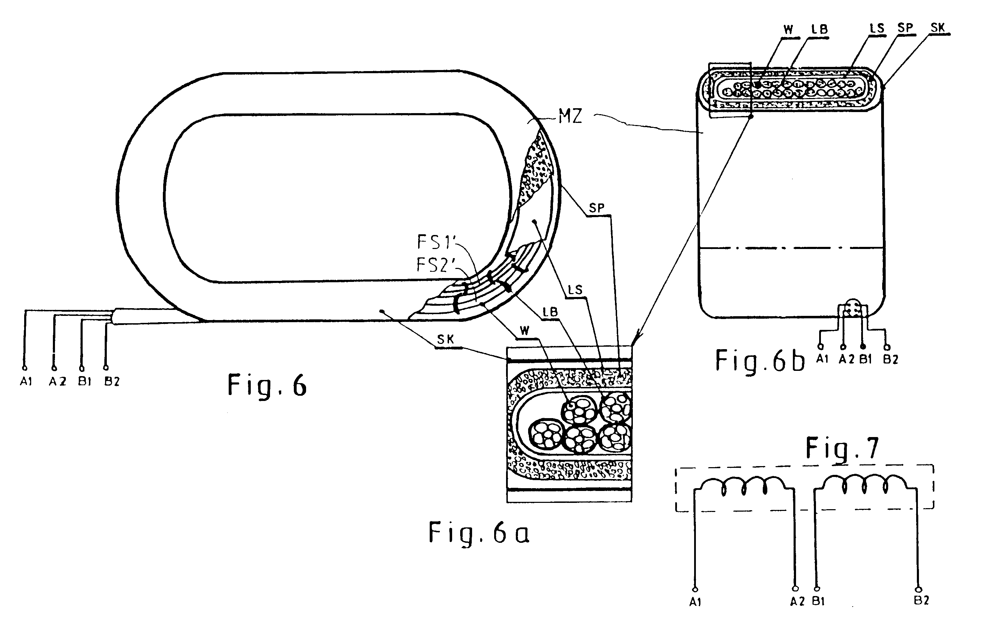

Fig. 6 zeigt eine hohl-zylindrische Feldspulenausgestaltung, bei der die beiden Spulenanordnungen (FS1', FS2') aus dem Litzenkabel (W) direkt aufeinander gelegt sind. Der innere Aufbau ist aus der teilweise geöffneten Darstellung, aus dem Querschnitt Fig. 6b und dem vergrößerten Querschnitt Fig. 6a zu ersehen. Die Kabel (W) sind mit einer Ummantelung (LB), z.B. aus Leinenband, versehen, die der Spule ihre hohlzylindrische oder hohl-flachzylindrische Form gibt und ihr eine Flexibilität verleiht. Die gesamte Zylinderspule ist mit einer Textilummantelung (LS), darauf einer Schaumstoff- oder Schwammschicht (SP) und mit einer Kunststoff- oder Naturstoffhülle (SK) umgeben. Die flexible Ausführung der Spule ist sehr praktisch in der Handhabung und beim Transport, da ihre Gestalt den Bedürfnissen im Einzelfall anpaßbar ist.

Fig. 7 zeigt das elektrische Anschlußschema und die additive Magnetfeldlage.

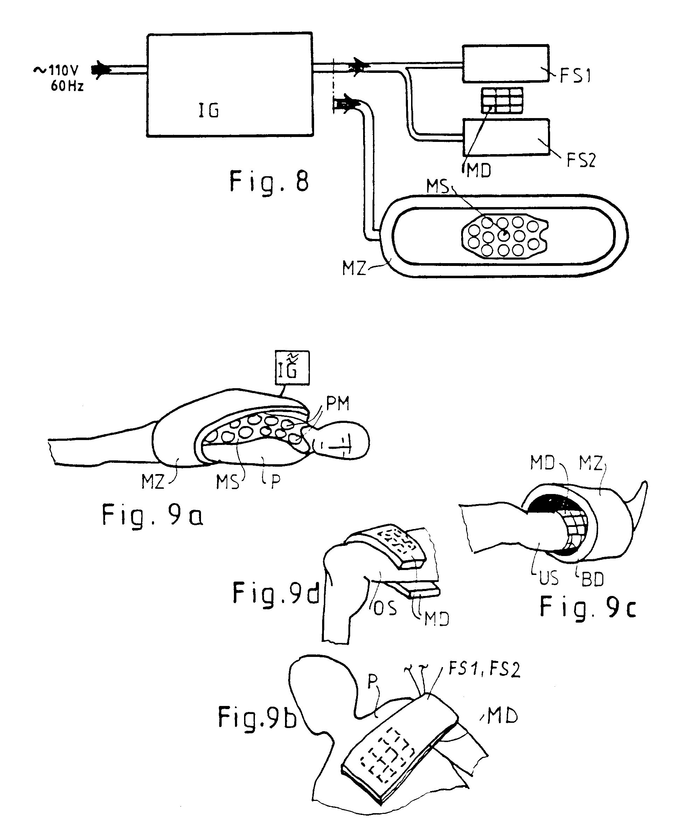

Fig. 8 zeigt eine Zusammenstellung des Impulsgenerators (IG) mit den wahlweisen anschließbaren Feldspulenanordnungen (FS1, FS2; MZ) und der Magnet-Decke (MD) oder der Magnetschürze (MS), rein schematisch.

Fig. 9a zeigt die Anordnung einer Magnetschürze (MS) auf einem Patienten (P); die Magnetspule ist durch die flexiblen Anordnungen (FS1, FS2) gegeben, die auf die Magnetdecke (MD) aufgelegt ist.

Fig. 9b zeigt die Anordnung einer Bandage (BM) mit einer Magnetdecke (MD) eines Unterschenkels (US) und die umgebende Zylinderspule (MZ).

Fig. 9d zeigt eine lose Anordnung einer Magnetdecke (MD) auf einem Oberschenkel (OS); die Feldspule ist nicht dargestellt.

Fig. 9a bis 9d sind lediglich Anwendungs- und Ausgestaltungsbeispiele. Eine Anpassung an besondere Anwendungsfälle sowie an Tierkörper ist fachmännisch möglich. Statt der flexiblen Spulen, die besonders angenehm in der Anwendung sind, lassen sich auch feste Spulen verwenden. Die Spulen können auch in Kleidungsstücke eingearbeitet werden ebenso wie die Magnete. Die für die Massage-Schürze oder Massage-Decke verwandten Permanentmagnete lassen sich auch durch in sich starre, jedoch gegeneinander flexibel gehaltene Elektromagnete, die aus Spulen bestehen, verwenden, die mit Gleichstrom durchflossen sind; diese benötigen dann jedoch eine Betriebsbestromung.

Eine weitere Ausgestaltung der Permanentmagnet-Decke oder -Schürze ist dadurch gegeben, daß sie aus einer gerastert geprägten Platte aus magnetisiertem Magnetgummi gebildet wird. Diese kann bedarfsweise mit einer textilen und/oder elastischen Hülle versehen sein. Die Abmessung der Magentplättchen ist bezüglich der Kantenlängen oder des Durchmessers im Bereich von 0,5 - 5 cm als günstig gefunden worden. Die Schwenkbewegung der Plättchen im Niederfrequenzfeld der Zylinderspule, deren Feld senkrecht zur Plättchenmagnetisierung orientiert ist, oder die Achsialbewegung der Plättchen, wenn deren Felder parallel zum Niederfrequenzfeld der elastischen Spulenanordnung gerichtet sind, wirkt zusammen mit den richtungsorientierten Gefäßwandungen der Blut- und Lympfgefäße wie eine Schlauchpumpe, nach Art einer Peristaltik, die Blutzirkulation fördernd und drainierend, wodurch auch im Anwendungsgebiet der Sportübungsvor- und nachbereitung vorteilhafte Wirkungen gefunden wurden.

## Patentansprüche

1. Behandlungsvorrichtung für einen menschlichen oder tierischen Körper (P) oder Körperteil (US, OS) mit einer Feldspulenanordnung (FS1, FS2; MZ), die mit einem netzgespeisten, niederfrequenten Impulsstromgenerator (IG) verbunden ist und in der ein Magnetplättchen (PM) elastisch gehalten angeordnet ist, so daß es in dem niederfrequenten Magnetfeld der Feldspulenanordnung (FS1, FS2; MZ) oszilliert,
dadurch gekennzeichnet, daß mehrere Magnetplättchen (MP) gegeneinander verschwenkbar in einer Magnetdecke (MD) oder einer körpergerecht gestalteten Magnetschürze (MS) gehalten sind und zwischen den Magnetplättchen (MP) elastisches Material eingelagert ist, durch das Nähte (SE) hindurchgenäht sind, und daß die Feldspulenanordnung (FS1, FS2; MZ) aus nur einer hohlzylindrischen oder abgeflacht-hohlzylindrischen Feldspule (MZ) oder aus mehreren Spulenabschnitten (FA, FB, FC; FS1, FS2) besteht, die nebeneinander angeordnet sind und an den einzigen Impulsstromgenerator (IG) gleichsinnig wirkend angeschlossen sind.

2. Behandlungsvorrichtung nach Anspruch 1, dadurch gekennzeichnet, daß die Spulenabschnitte (FA, FB, FC) in Spiralform ausgebildet nebeneinander angeordnet sind.

3. Behandlungsvorrichtung nach Anspruch 1 oder 2, dadurch gekennzeichnet, daß die Feldspule (MZ) oder Feldpsulenabschnitte (FA, FB, FC; FS1, FS2) aus Leitern (W) bestehen, die mit einer flexiblen Umhüllung (LB) umschlossen sind, die diese in ihrer Lage fixiert.

4. Behandlungsvorrichtung nach Anspruch 3, dadurch gekennzeichnet, daß die flexible Umhüllung (LB) von einer textilen Schicht (LS) umschlossen ist, auf der eine Schaumstoffschicht (SP) aufliegt, über die eine flexible Kunst- oder Naturstoffhülle (SK) gelegt ist.

5. Behandlungsvorrichtung nach einem der vorstehenden Ansprüche, dadurch gekennzeichnet, daß der Impulsstromgenerator (IG) einen Oszillator (A) enthält, der mit einer vorgebbaren Folgefrequenz von 0,5 - 30 Hz sinus- oder halbsinusförmiger Netz-Wechselspannung mit einer Frequenz von z.B. 60 Hz abgibt.

6. Behandlungsvorrichtung nach einem der vorstehenden Ansprüche 1 bis 4, dadurch gekennzeichnet, daß der Impulsgenerator (IG) einen Oszillator (A) enthält, der Rechteckspannungsimpulse mit einer vorgebbaren Frequenz von 1 - 1000 Hz liefert.

7. Behandlungsvorrichtung nach einem der Ansprüche 5 oder 6, dadurch gekennzeichnet, daß die Ausgangsspannung des Impulsgenerators (IG) einstellbar ist.

8. Behandlungsvorrichtung nach Anspruch 5, dadurch gekennzeichnet, daß die Netzwechselspannung durch einen Triac (TRI) im Takte des ersten Oszillators (A) gesteuert einem Stelltransformator (TR1) zugeführt wird, dessen Ausgang über eine Diode (D) oder einen Schalterkontakt (SW2) an den Ausgang (CO) des Impulsgenerators (IG) geführt ist.

9. Behandlungsvorrichtung nach Anspruch 6, dadurch gekennzeichnet, daß die Netzwechselspannung über einen Stufentransformator (TR1) einem Gleichrichter (G) zugeführt ist und die da entstehende Gleichspannung durch elektronische Schalteranordnungen (TY) gepulst an den Ausgang (CO) des Impulsgenerators (IG) geführt ist, wobei die Schalteranordnung (TY) aus zwei Schaltergruppen besteht, die zeitlich komplementär von einem zweiten Oszillator (IC2) über einen Rechteckformer (IC3) angesteuert sind.

10. Behandlungsvorrichtung nach Anspruch 5 bis 9, dadurch gekennzeichnet, daß der Impulsgenerator (IG) die beiden Oszillatoren (A, B) enthält, die durch einen Betriebsartschalter (SW3) wahlweise einschaltbar sind.

## Claims

1. Treatment appliance for a human or animal body (P) or body part (US, OS), comprising a field coil arrangement (FS1, FS2; MZ) which is connected to a mains-operated, low-frequency power pulse current generator (IG) and in which a small magnetic plate (PM) is resiliently mounted, causing the plate to oscillate in the low-frequency magnetic field of the field coil arrangement (FS1, FS2; MZ),
characterised in that a plurality of small magnetic plates (MP) are held swivellable relative to one another in a magnetic cover (MD) or a magnetic apron (MS) shaped to fit the body and interposed between the magnetic plates (MP) is elastic material through which seams (SE) are sewn, and in that the field coil arrangement (FS1, FS2; MZ) consists of only one hollow cylindrical or flattened hollow cylindrical field coil (MZ) or of a plurality of coil sections (FA, FB, FC; FS1, FS2) which are arranged alongside one another and are linked to the single power pulse current generator (IG) so as to operate in the same direction.

2. Treatment appliance according to claim 1, characterised in that the coil sections (FA, FB, FC) are designed in spiral form and arranged alongside one another.

3. Treatment appliance according to claim 1 or 2, characterised in that the field coil (MZ) or field coil sections (FA, FB, FC; FS1, FS2) consist of conductors (W) surrounded by a flexible sheath (LB) which fixes them in position.

4. Treatment appliance according to claim 3, characterised in that the flexible sheath (LB) is surrounded by a textile layer (LS) on which a layer of foamed material (SP) rests, over which is laid a flexible envelope (SK) of plastic or natural material.

5. Treatment appliance according to one of the preceding claims, characterised in that the power pulse current generator (IG) contains an oscillator (A) which at a definable repetition rate of 0.5 to 30 Hz emits sinusoidal or half-sinusoidal a.c. supply voltage at a frequency of, for example, 60 Hz.

6. Treatment appliance according to one of the preceding claims 1 to 4, characterised in that the pulse generator (IG) contains an oscillator (A) which delivers square-wave voltage pulses at a definable frequency of 1 - 1000 Hz.

7. Treatment appliance according to one of claims 5 or 6, characterised in that the output voltage of the pulse generator (IG) is adjustable.

8. Treatment appliance according to claim 5, characterised in that the a.c. supply voltage is supplied, under the control of a triac (TRI) in phase with the first oscillator (A), to a variable transformer (TR1) the output of which passes via a diode (D) or a switch contact (SW2) to the output (CO) of the pulse generator (IG).

9. Treatment appliance according to claim 6, characterised in that the a.c. supply voltage is supplied via a tapped transformer (TR1) to a rectifier (G) and the direct voltage there produced is sent, pulsed by electronic switch assemblies (TY), to the output (CO) of the pulse generator (IG), the switch assembly (TY) comprising two switch groups activated in chronologically conplementary manner by a second oscillator (IC2) via a square-wave generator (IC3).

10. Treatment appliance according to claims 5 to 9, characterised in that the pulse generator (IG) contains the two oscillators (A, B), which can be switched on as required using an operating mode selector (SW3).

## Revendications

1. Dispositif de traitement du corps (P) ou d'un membre du corps humain ou animal (US, OS), avec un agencement de bobines de champ (FS1, FS2; MZ), relié à un générateur de courant pulsé à fréquence basse (IG), alimenté par le réseau, et dans lequel une plaquette magnétique PM) est disposée, maintenue élastiquement, de sorte qu'elle oscille dans le champ magnétique à basse fréquence de l'agencement de bobines de champ FS1, FS2; MZ),
caractérisé par le fait
que plusieurs plaquettes magnétiques (MP) sont maintenues, pivotantes par rapport les unes aux autres, dans une couverture magnétique (MD) ou un tablier magnétique (MS), adapté au corps, et qu'une matière élastique est cousue entre les plaquettes magnétiques (MP), à l'aide de coutures (SE), et que l'agencement de bobines de champ (FS1, FS2; MZ) est composé d'une seule bobine de champ cylindrique creuse ou cylindrique creuse, aplatie (MZ) ou de plusieurs segments de bobine (FA, FB, FC; FS1, FS2), disposés les uns à côté des autres et raccordés, agissant dans le même sens, à l'unique générateur de courant pulsé (IG).

2. Dispositif de traitement selon revendication 1,
caractérisé par le fait
que les segments de bobine (FA, FB, FC) sont disposés les uns à côté des autres, en forme de spirale.

3. Dispositif de traitement selon revendication 1 ou 2,
caractérisé par le fait
que la bobine de champ (MZ) ou les segments de bobine de champ (FA, FB, FC; FS1, FS2) consistent en conducteurs (W), entourés d'une gaine flexible (LB), les maintenant dans leur position.

4. Dispositif de traitement selon revendication 3,
caractérisé par le fait
que la gaine flexible (LB) est entourée d'un revêtement textile (LS), sur lequelle une couche de matière moussée (SP) est appliquée, le tout étant recouvert d'une enveloppe flexible en matière naturelle ou synthétique (SK).

5. Dispositif de traitement selon l'une des revendications précédentes,
caractérisé par le fait
que le générateur de courant pulsé (IG) comprend un oscillateur (A), qui, avec une fréquence sérielle prédéterminable de 0,5 à 30 Hz, émet une tension alternative du réseau sinusoïdale ou semisinusoïdale d'une fréquence de, par exemple, 60 Hz.

6. Dispositif de traitement selon l'une des revendications 1 à 4,
caractérisé par le fait
que le générateur d'impulsions (IG) comprend un oscillateur, qui délivre des impulsions rectangulaires avec une fréquence prédéterminable de 1 à 1000 Hz.

7. Dispositif de traitement selon revendication 5 ou 6,
caractérisé par le fait
que la tension de sortie du générateur d'impulsions (IG) est réglable.

8. Dispositif de traitement selon revendication 5,
caractérisé par le fait que
la tension alternative du réseau, commandée par un triac (TRI) selon le rythme du premier oscillateur (A), est conduite à un transformateur régulateur (TR1), dont la sortie est menée à la sortie (CO) du générateur d'impulsions (IG), par l'intermédiaire d'une diode (D) ou d'un contact de commutateur (SW2).

9. Dispositif de traitement selon revendication 6,
caractérisé par le fait
que la tension alternative du réseau est conduite à un redresseur (G), par l'intermédiaire d'un transformateur à prises (TR1) et que la tension continue en résultant est conduite, pulsée par un circuits de commutation électronique (TY), à la sortie (CO) du générateur d'impulsions, le circuit de commutation (TY) étant composé de deux groupes de commutateurs, qui sont excités, complémentairement sur le plan temporel, par un deuxième oscillateur (IC2), par l'intermédiaire d'un formateur d'impulsions rectangulaires (IC3).

10. Dispositif de traitement selon les revendications 5 à 9,
caractérisé par le fait
que le générateur d'impulsions (IG) comprend les deux oscillateurs (A, B), qui peuvent être connectés, au choix, à l'aide d'un commutateur sélecteur de mode de service (SW3).
